# EUROPEAN PATENT APPLICATION

(11) **EP 2 443 989 A1**
(43) Date of publication of application: **25.04.2012**
(21) Application number: 11250858.5
(22) Date of filing: 19.10.2011
(51) Int. Cl.: A61B 1/12, A61B 1/00, A61B 1/313

(54) **Cleaner for endoscope**

(30) Priority: 20.10.2010 US 394810 P; 26.09.2011 US 245025
(71) Applicant: Tyco Healthcare Group LP, New Haven, CT 06511 (US)
(72) Inventor: Ma, Yong, Cheshire, CT 06410 (US); Power, James M., Madison, CT 06443 (US)
(74) Representative: Soames, Candida Jane

(57) **Abstract**

A surgical scope for viewing a surgical site of a patient including an elongated tube, a viewing lens having an outer surface positioned at the distal portion of the scope, and a lens cleaner positioned at the distal portion and translatable between a first position and a second position. The lens cleaner includes an arcuate wiper having a complementary shape to the shape of the lens. An actuator is spaced proximally of the wiper and actuable to translate the wiper along the outer surface of the lens.

## Description

### BACKGROUND

This application claims priority from provisional application serial no. 61/394,810, filed October 20, 2010, the entire contents of which are incorporated herein by reference.

### Technical Field

The present disclosure relates to a cleaning apparatus configured to remove debris from the lens of a minimally invasive viewing instrument.

### Background of Related Art

Minimally invasive surgery has become increasingly popular in recent years. Minimally invasive surgery eliminates the need to cut a large incision in a patient, thereby reducing discomfort, recovery time, and many of the deleterious side effects associated with traditional open surgery. Minimally invasive viewing instruments, e.g., laparoscopes and endoscopes, facilitate the viewing of internal tissues and/or organs.

Laparoscopic surgery involves the placement of a laparoscope in a small incision in the abdominal wall of a patient to view the surgical site. Endoscopic surgery involves the placement of an endoscope in a naturally occurring orifice, e.g., mouth, nose, anus, urethra, and vagina to view the surgical site. Other minimally invasive surgical procedures include video assisted thoracic surgery and cardiovascular surgery conducted through small incisions between the ribs. These procedures also utilize scopes to view the surgical site.

A typical minimally invasive viewing instrument, e.g., a laparoscope or an endoscope, includes a housing, an elongated lens shaft extending from one end of the housing, and a lens that is provided in the distal end of the lens shaft. A camera viewfinder extends from the other end of the housing. A camera is connected to the housing and transmits images of the surgical field viewed through the lens to a monitor on which the images are displayed. During a surgical procedure, the distal end portion of the lens shaft is extended into the patient, while the proximal end portion of the lens shaft, the housing and the camera viewfinder remain outside the patient. In this manner, the laparoscope/endoscope is positioned and adjusted to view particular anatomical structures in the surgical field on the monitor.

During insertion of an endoscope or a laparoscope into the body and during the surgical procedure, debris, e.g., organic matter and moisture, may be deposited on the lens of the endoscope. The buildup of debris on the lens impairs visualization of the surgical site, and often necessitates cleaning of the lens.

### SUMMARY

Disclosed herein is a surgical instrument for use in a minimally invasive surgery.

The surgical instrument includes a surgical scope for viewing a surgical site of a patient comprising an elongated tube having proximal and distal portions, a viewing lens having an outer surface positioned at the distal portion of the scope, and a lens cleaner positioned at the distal portion and translatable between a first position and a second position. The lens cleaner includes an arcuate wiper having a complementary shape to the shape of the lens. An actuator is spaced proximally of the wiper and is actuable to translate the wiper along the outer surface of the lens.

The lens cleaner in some embodiments includes a band and a wiping structure extending from the band.

The instrument can include an elongated actuating mechanism operatively connecting the wiper to the actuator. In one embodiment, the instrument includes a bar including a rack, and the bar is translatable along the longitudinal axis of the scope to effect pivoting of the arcuate wiper.

In preferred embodiments, the arcuate wiper includes a surface that is in operative contact with the outer surface of the endoscope lens. Such surface in one embodiment is absorbent. Such surface can include bristles, a fabric and/or a cloth. In some embodiments, the surface of the arcuate wiper has a texture.

In some embodiments, a plurality of elongated members are positioned inside the scope and connected at distal ends to the wiper to pivot the wiper mechanism over the lens.

The instrument can include an attachment member that is operatively connectable to the wiper to facilitate cleaning of a substantially flat lens. The attachment member may be formed from a material having compressible properties, e.g., a sponge-like material.

In some embodiments, the wiper includes an elastic strip that conforms to the shape of the lens.

In another aspect, a lens cleaner for use with a surgical scope is provided comprising an arcuate band and an arcuate wiper extending inwardly from the band, the wiper including a material for contacting and moving along the lens of the scope to remove debris as the wiper is moved from a first to a second position.

In some embodiments, the wiper includes a surface that is absorbent. The wiper can include bristles, a fabric and/or a cloth. In some embodiments, the wiper includes an elastic strip that conforms to a rounded shape of the lens. An attachment member can be provided that is operatively connectable to the wiper to facilitate cleaning of a substantially flat lens.

These and other features of the present disclosure will be more fully described with reference to the appended figures.

### BRIEF DESCRIPTION OF THE DRAWINGS

By way of description only, embodiments of the present disclosure will be described with reference to the accompanying drawings, in which:

Figure 1 is a perspective view of a scope with a lens cleaner in accordance with an embodiment of the present disclosure;

Figure 2 is an enlarged view of one embodiment of an actuator for actuating the lens cleaner;

Figure 2A is a view similar to Figure 2 illustrating the actuator in an actuated position;

Figure 3 is an enlarged perspective view illustrating the lens cleaner of Figure 1 in a first position;

Figure 3A is an enlarged perspective view similar to Figure 3 illustrating the lens cleaner moved to a second position across the lens of the scope;

Figure 4 is a perspective view of one embodiment of the lens cleaner;

Figure 5 is a cross-sectional view taken along line 5-5 of Figure 4;

Figure 6 is a perspective view of another embodiment of the lens cleaner;

Figure 7 is a cross-sectional view taken along line 7-7 of Figure 6;

Figure 8 is a perspective view of yet another embodiment of the lens cleaner;

Figure 9 is a cross-sectional view taken along line 9-9 of Figure 8;

Figure 10 is a perspective view of a scope with a lens cleaner in accordance with an alternate embodiment of the present disclosure;

Figure 11 is an enlarged perspective view of an actuator mechanism for moving the lens cleaner;

Figure 12A is a perspective view of one embodiment of an attachment member for use with a wiper;

Figure 12B is a perspective view of the attachment member of Figure 12A coupled to a wiper;

Figure 12C is a perspective view of another embodiment of an attachment member for use with a wiper; and

Figure 12D is a perspective view of the attachment member of Figure 12C coupled to a wiper.

### DETAILED DESCRIPTION

Particular embodiments of the present disclosure will be described herein with reference to the accompanying drawings. In the figures and in the description that follows, in which like reference numerals identify similar or identical elements, the term "proximal" will refer to the end of the apparatus that is closer to the operator during use, while the term "distal" will refer to the end that is further from the operator during use.

An endoscope typically includes an endoscope housing or body which can be rigid or flexible, depending on its surgical application. A camera viewfinder, e.g. an eyepiece, is located at a proximal (imaging) end of the scope housing. A lens is provided at the distal end of the scope body.

In typical use of the endoscope, the viewfinder is adapted to sight images of a surgical field in the patient, e.g. an abdominal cavity, thoracic cavity, etc., as the position of the scope is adjusted to view a particular anatomical structure or structures in the surgical field. The camera is adapted to receive images of the surgical field sighted through the lens and transmit the images to an external monitor that is connected to the camera and on which the images of the surgical field are displayed. That is, a visual display device is operatively connected to the eyepiece to convert the optical signal into a video signal to produce a video image on the monitor (or for storage on select media). Accordingly, the monitor enables a surgical team to view the anatomical structure or structures in the surgical field inside the patient as the surgical procedure is carried out using minimally invasive or endoscopic surgical instruments. Throughout the surgical procedure, biological tissue or matter has a tendency to contact and build up on the lens of the scope. This tends to obscure the images of the surgical field as they are displayed on the monitor.

The endoscope of the present disclosure includes a wiper movable to clean the lens of the scope during the surgical procedure to maintain a clear image without having to remove the scope from the patient's body.

Note the endoscope can be an optical scope or an electronic scope which contains video signal wires that can be connected to a video monitor.

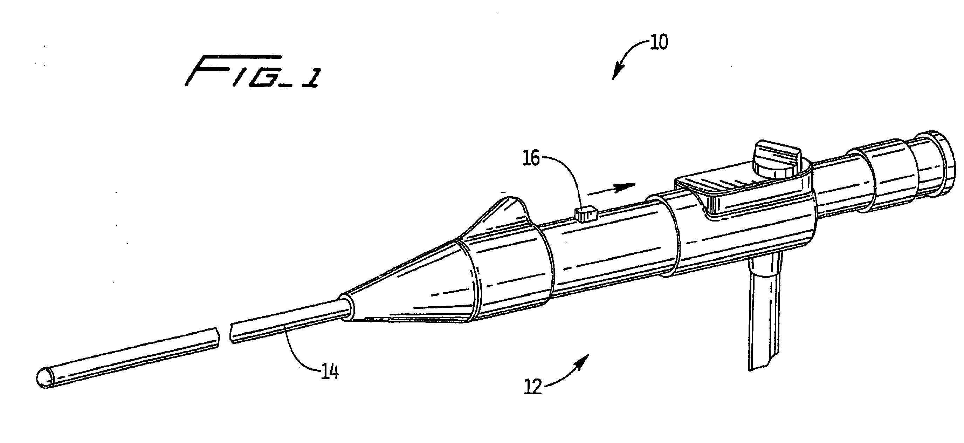

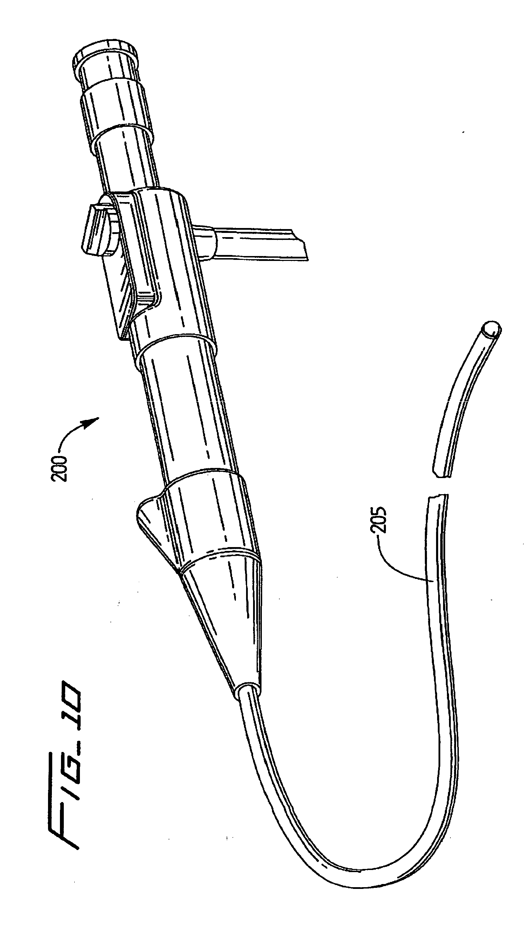

Referring initially to Figure 1, a minimally invasive viewing instrument, e.g. an endoscope, is designated generally by reference numeral 10. The instrument 10 generally includes a handle portion 12 and an elongated tube 14 extending therefrom and having a generally annular cross-sectional configuration. The tube 14 may be formed from a substantially rigid, semi-rigid, or a flexible material. Figure 1 illustrates a rigid scope having a rigid tube 14. Figure 10 illustrates a flexible scope 200 having a flexible tube 205. The lens cleaners of the present disclosure can be utilized with flexible, semi-rigid or rigid scopes.

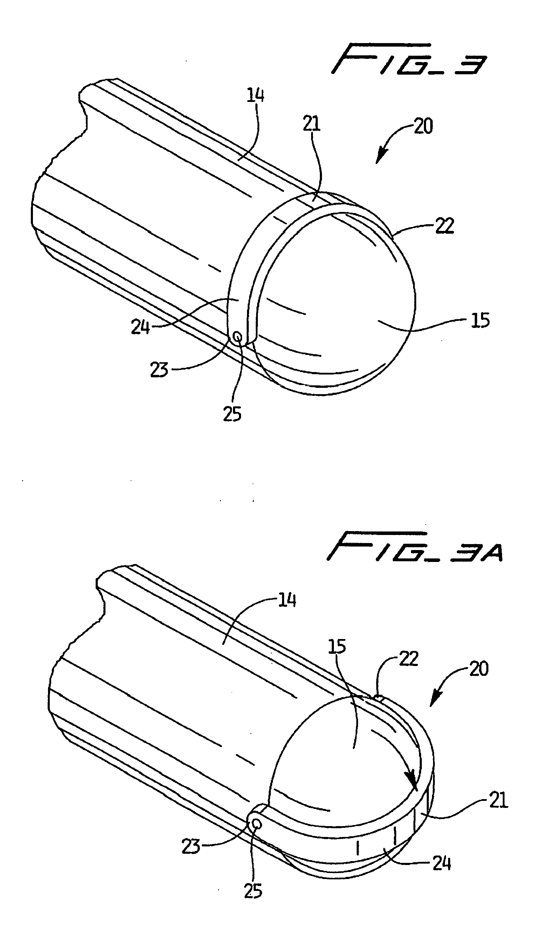

A first embodiment of the lens cleaner 20 is illustrated in Figures 3 and 3A. Lens cleaner 20 includes an arch shaped wiper 21 which is configured to pass across the lens 15 of the endoscope 10. The wiper 21 is attached to an actuating mechanism, such as a rod or wire, at its ends 22, 23 as described below. A pin 25 at each end 22, 23 can be utilized to attach the wiper 21 to the actuating mechanism. Thus, wiper 21 preferably has two anchor points, spaced about 180° degrees apart in the illustrated embodiment. Other spacings are also contemplated. As shown, the wiper 21 has a curve or arch shape to complement the curved shape of the endoscope lens 15. This arcuate shape of the wiper 21 also enables it to be kept out of the field of view of the lens during the surgical procedure.

In the initial position of the wiper 21 illustrated in Figure 3, it is spaced from the scope lens 15 so as not to obstruct visualization of the surgical site. To clean debris or other particles form the lens 15 during a surgical procedure, the wiper 21 is rotated across the outer surface of the lens 15 as shown by the arrow of Figure 3A. Note the arch shape of the wiper 21 enhances cleaning as it conforms to the dome shape of the lens 15. In the illustrated embodiment, the arch extends about 180 degrees, although arches of other degrees are also contemplated.

The wiper mechanism 21 includes an arcuate band 24 and a wiping structure on at least a portion of the internal surface thereof for directly contacting the outer surface of the lens 15. In the embodiment of Figure 3, the wiping structure is integral with the band 24, i.e. the band is composed of a material suitable for wiping the lens 15. That is, in this embodiment, band 24 is formed from a material or may have a surface that facilitates cleaning of the lens. For example, the band 24 may have a texture that is abrasive to facilitate removal of debris from the lens surface as it passes over the outer surface.

A torsion spring can be utilized to bias the wiper 21 toward a retracted state, i.e. removed from the viewing field of the lens 15 of the endoscope 10 so as not to obstruct the view (see Figure 3). The torsion spring can be associated with the actuating mechanism or the actuator (discussed below) to bias the actuating mechanism to maintain the wiper 21 in the retracted state.

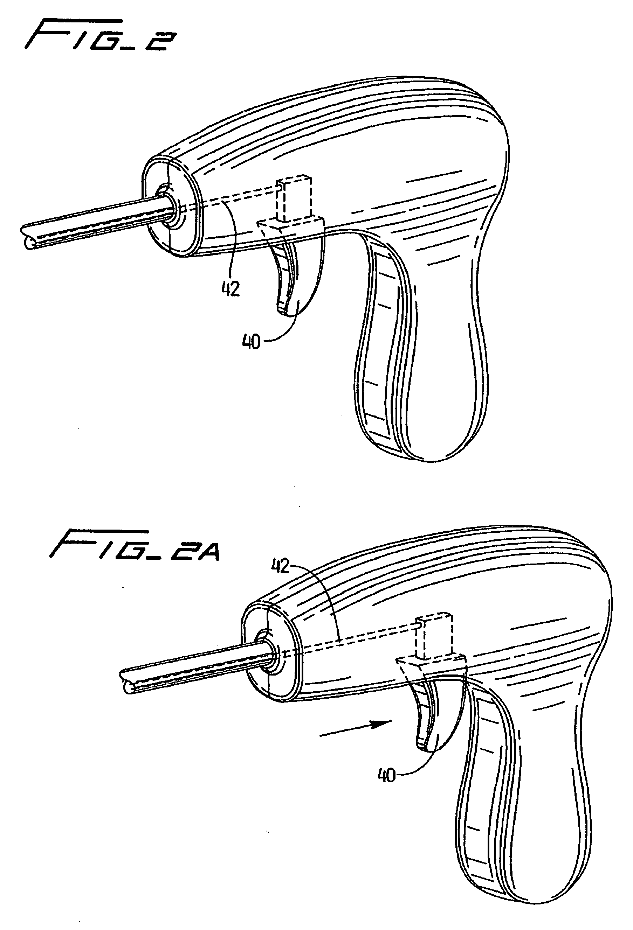

Actuation of the band 24 of wiper 21 may be achieved by an actuation mechanism in the form of a drawstring, rod, wire, or other mechanism which can be actuated at a proximal end of the device. That is, the actuation mechanism would be positioned within the scope tube 14 and attached at a distal end to the band 24 and at a proximal end to an actuator. The actuator can be in the form of a trigger, for example, as shown in Figures 2 and 2A. In the initial position of Figure 2, trigger 40 is at an at rest position and the wiper 21 is in the position of Figure 3. To move the wiper 21 across the lens 15, trigger 40 is pulled in the direction of the arrow of Figure 2A, thereby pulling the actuating rod 42, operatively connected thereto, rearwardly to pivot the wiper 21 across the lens 15. The actuating rod 42 is preferably split in order to connect to both pivot ends 22, 23 of the wiper 21. Alternatively, two separate rods can be operatively connected to trigger 40, one connected to first end 22 and the second rod connected to second end 23 of wiper 21. Wires or other elongated actuating mechanisms operatively connecting the wiper blade 21 to the actuator can also be utilized.

The trigger mechanism 40 is preferably biased to the non-actuated position of Figure 2 such that upon release of the trigger, it returns to the position of Figure 2, thereby moving the wiper 21 back to its initial position of Figure 3. In this manner, trigger 40 can be repeatedly actuated during the surgical procedure to actuate the wiper 21 to clean the lens when desired by the user.

Other actuators are also contemplated such as a rotating knob or a sliding knob 16 as shown in Figure 1 which is movable in the direction of the arrow to retract the actuating mechanism to pivot the wiper 21. Also contemplated is a motor for moving the actuating mechanism. The motor can provide for select actuation of the wiper 21 when desired by the user or alternatively provide for automatic intermittent movement of the wiper 21 across the lens 15 thereby creating a "blink camera."

As an alternative to the rod 42, a wire, drawstring(s) or other actuating mechanism can be operatively connected at a proximal end to an actuator and at the opposite end to the band of the wiper. Preferably, two wires, two drawstrings, etc. would be provided so the band is operatively connected to the actuator at two anchor points.

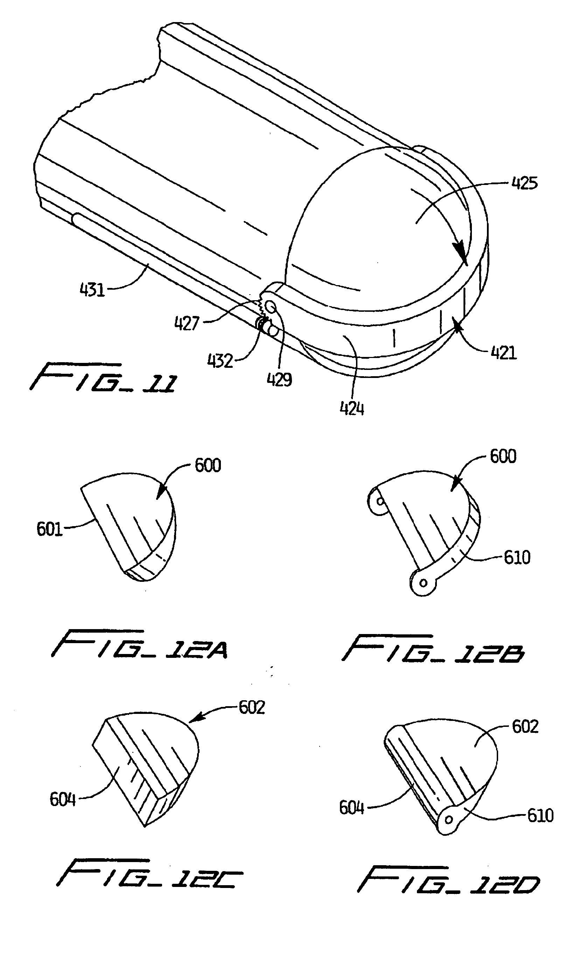

Another actuating mechanism is shown in Figure 11 in the form of a rack and pinion. The band 424 of wiper 421 has two connection ends forming two pivot points 429 (only one is shown in Figure 11), each with a gear 427. The actuating mechanism has a plurality of longitudinally extending teeth on rack 432 formed on the distal end of actuating bar 431. Two actuating bars 431 are provided, each including a rack 432. The rack teeth intermesh with the teeth of gears 427. As actuating bar 431 is pulled rearwardly by an actuator such as an actuator of the type described herein with respect to the other embodiments (e.g. a trigger or sliding knob), the actuating mechanism is retracted causing the band 424 to pivot across scope lens 425. The actuator can be coupled to the proximal end of the bar 432 to facilitate translation of the bar. The various wiping structures described herein, as well as other structures, can be provided on wiper 421.

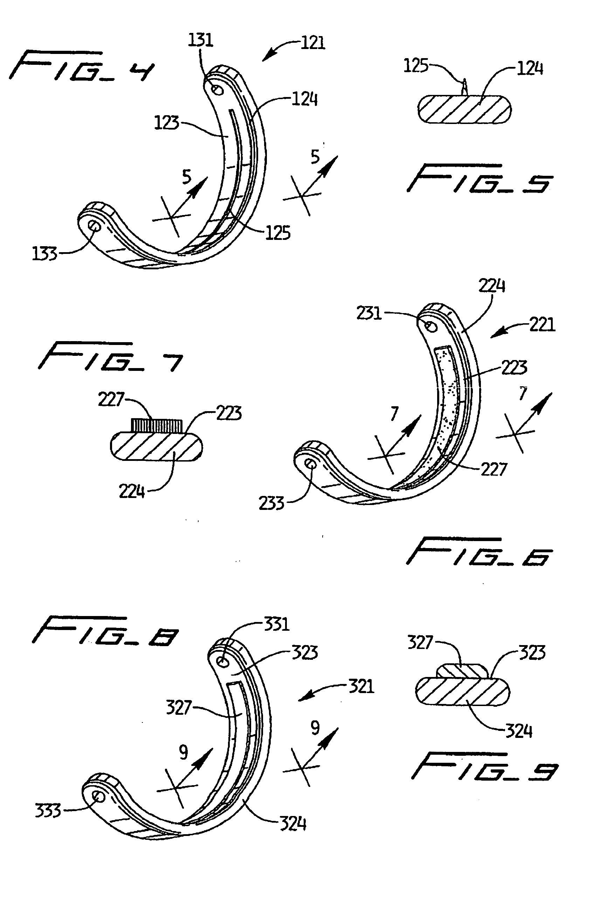

Figures 4-9 illustrate alternate embodiments of the wiping structure of the lens cleaners of the present disclosure to clean the lens. In the embodiment of Figure 4, wiper 121 includes a band 124 with a wiping structure in the form of a soft flexible projection 125 for contact with the lens 15. As shown, projection 125 extends inwardly from an inner surface 123 of wiper 121 to engage the lens 15 of the endoscope. Projection 125 extends in an arch and as shown preferably occupies a small transverse area of the internal surface 123 of band 121. Although the projection 125 is shown substantially triangular in cross-section, other shapes are also contemplated. Openings 131, 133 connect the band 124 to an actuating mechanism such as the actuating mechanisms discussed above, to provide two pivot points to rotate the wiper 21 across the outer surface of the lens 15.

The wiper 221 of the embodiment of Figure 7 is identical to wiper 121 of Figure 4 except for the wiping structure. Thus, wiper 221 has a band 224 with an internal surface 223 and two anchor points forming two pivot connections 231, 233 as in the other embodiments described herein. Wiper 221 however has a set of bristles 227 extending inwardly from band 223. Bristles 227 extend in an arc and preferably occupy a substantial portion of the internal surface 223 of band 224, although different size bristle areas are also contemplated. When the wiper 221 is moved across the lens 15 in the manner described above, the bristles 227 move across the lens 15 of the endoscope in a brush-like manner to clear particles from the lens 15.

Wiper 321 of the embodiment of Figure 8 is identical to wiper 121 of Figure 4 except for the wiping structure. Thus, wiper 321 has a band 324 with an internal surface 323 and two anchor points forming two pivot connections 331, 333. Wiper 321 however has a cloth material 327 extending inwardly from band 323. The cloth material can occupy a substantial portion of the internal surface of band 324 as shown, although different size cloths are also contemplated. When the wiper 321 is moved across the lens 15 in the manner described above, the cloth 327 wipes across the lens 15 of the endoscope to clear particles from the lens. The cloth can optionally include a textured surface. The cloth may have drying properties to remove moisture from the outer surface of the endoscope lens.

Different materials and/or devices may be coupled to the inner surface of the wipers described herein. The wiper may be formed from different materials. The wiper is adapted to compress against the outer surface of the endoscope lens as the wiper surface contacts and passes over the lens surface.

Although shown for use with a dome or curved endoscope lens, the lens cleaner of this disclosure can also be used with a flat lens, with the wiper shaped accordingly to contact and clean the scope lens as it is pivoted or moved thereover. The wiper can include a flexible strip having elastic properties. As the wiper is retracted and drawn across the surface of the lens, the flexible strip stretches and rubs against the surface of the lens to remove moisture and/or debris from the surface of the lens of the endoscope.

For use with differently shaped lenses e.g., a flat scope lens, an attachment member 600, 602 may be operatively coupled with wiper 610. As shown in Figs. 12A and 12C, the attachment members 600, 602 differ in shape. In particular, the attachment member 600 may be wedge shaped with an edge 601. The attachment member 602 may have a thicker end 604 which has a substantially planar surface. The attachment members 600, 602 are preferably formed from a compressible material having sponge-like properties. As the wiper 610 is translated across the lens of the scope, the wiper 610 compresses to conform to the flat shape of the surface of the lens.

Actuation of the wiper mechanisms disclosed herein may be achieved through mechanical or electro-mechanical means. In addition, the actuation may occur automatically, intermittently, and/or in response to certain conditions.

While several embodiments of the disclosure have been shown in the drawings and/or discussed herein, it is not intended that the disclosure be limited thereto, as it is intended that the disclosure be as broad in scope as the art will allow and that the specification be read likewise. Moreover, it is to be understood that the lens cleaners disclosed herein may be used with any instrument including a lens that is used during a minimally invasive surgical procedure. Therefore, the above description should not be construed as limiting, but merely as exemplifications of particular embodiments. Those skilled in the art will envision other modifications within the scope and spirit of the claims appended hereto.

The invention may be described by reference to the following numbered paragraphs:
1. A surgical scope for viewing a surgical site of a patient comprising an elongated tube having proximal and distal portions, a viewing lens having a an outer surface positioned at the distal portion of the tube, a lens cleaner positioned at the distal portion and translatable between a first position and a second position, the lens cleaner including an arcuate wiper having a complementary shape to the shape of the lens and an actuator spaced proximally of the wiper and actuable to translate the wiper along the outer surface of the lens.
2. The surgical scope of paragraph 1, wherein the actuator includes a trigger.
3. The surgical scope of paragraph 1, wherein the lens cleaner includes a band and a wiping structure extending from the band.
4. The surgical scope of paragraph 3, further comprising an elongated actuating mechanism operatively connecting the wiper to the actuator.
5. The surgical scope of paragraph 1, further including a bar including a rack, the bar being translatable along a longitudinal axis of the elongated tube to effect pivoting of the wiper.
6. The surgical scope of paragraph 1, wherein the wiper includes a surface that is in operative contact with the lens surface of the viewing lens, and wherein the surface of the wiper is absorbent.
7. The surgical scope of paragraph 1, wherein the wiper includes a surface that is in operative contact with the lens surface of the viewing lens, and wherein the surface of the wiper includes bristles.
8. The surgical scope of paragraph 1, wherein the wiper includes a surface that is in operative contact with the lens surface of the viewing lens, and wherein the surface of the wiper has a texture.
9. The surgical scope of paragraph 1, wherein the wiper includes a surface that is in operative contact with the lens surface of the viewing lens, and wherein the surface of the wiper is a fabric or a cloth.
10. The surgical scope of paragraph 1, further comprising a plurality of elongated members positioned inside the scope and operatively connected at distal ends to the wiper to pivot the wiper over the lens.
11. The surgical scope of paragraph 1, further comprising an attachment member that is operatively connectable to the wiper to facilitate cleaning of a substantially flat lens.
12. The surgical scope of paragraph 1, wherein the wiper includes an elastic strip that conforms to the shape of the lens.
13. A lens cleaner for use with a surgical scope, the lens cleaner comprising an arcuate band and an arcuate wiper extending inwardly from the band, the wiper including a material for contacting and moving along the lens of the scope to remove debris as the wiper is moved from a first to a second position.
14. The surgical scope of paragraph 13, wherein the wiper includes a surface that is absorbent.
15. The surgical scope of paragraph 13, wherein the wiper includes bristles.
16. The surgical scope of paragraph 13, wherein the wiper includes a surface having a texture.
17. The surgical scope of paragraph 13, wherein the wiper includes a fabric or a cloth.
18. The surgical scope of paragraph 13, wherein the wiper includes an elastic strip that conforms to the shape of the lens.
19. The surgical scope of paragraph 13, further comprising an attachment member that is operatively connectable to the wiper to facilitate cleaning of a substantially flat lens.

## Claims

1. A surgical scope for viewing a surgical site of a patient comprising:
an elongated tube having proximal and distal portions;
a viewing lens having an outer surface positioned at the distal portion of the tube;
a lens cleaner positioned at the distal portion and translatable between a first position and a second position, the lens cleaner including an arcuate wiper having a complementary shape to the shape of the lens; and
an actuator spaced proximally of the wiper and actuable to translate the wiper along the outer surface of the lens.

2. The surgical scope of claim 1, wherein the actuator includes a trigger.

3. The surgical scope of claim 1 or 2, wherein the lens cleaner includes an arcuate band and a wiping structure extending from the band.

4. The surgical scope of any of claims 1-3, further comprising an elongated actuating mechanism operatively connecting the wiper to the actuator.

5. The surgical scope of any of claims 1-4, further including a bar including a rack, the bar being translatable along a longitudinal axis of the elongated tube to effect pivoting of the wiper.

6. The surgical scope of any of claims 1-5, wherein the wiper includes a surface that is in operative contact with the lens surface of the viewing lens, and wherein the surface of the wiper is absorbent.

7. The surgical scope of any of claims 1-6, wherein the wiper includes a surface that is in operative contact with the lens surface of the viewing lens, and wherein the surface of the wiper includes bristles.

8. The surgical scope of any of claims 1-6, wherein the wiper includes a surface that is in operative contact with the lens surface of the viewing lens, and wherein the surface of the wiper has a texture.

9. The surgical scope of any of claims 1-6, wherein the wiper includes a surface that is in operative contact with the lens surface of the viewing lens, and wherein the surface of the wiper is a fabric or a cloth.

10. The surgical scope of any of claims 1-9, further comprising a plurality of elongated members positioned inside the scope and operatively connected at distal ends to the wiper to pivot the wiper over the lens.

11. The surgical scope of any of claims 1-10, further comprising an attachment member that is operatively connectable to the wiper to facilitate cleaning of a substantially flat lens.

12. The surgical scope of any of claims 1-11, wherein the wiper includes an elastic strip that conforms to the shape of the lens.
